# EUROPEAN PATENT APPLICATION

(11) **EP 4 521 095 A1**
(43) Date of publication of application: **12.03.2025**
(21) Application number: 23196008.9
(22) Date of filing: 07.09.2023
(51) Int. Cl.: G01N 1/40, G01N 33/18

(54) **METHOD AND APPARATUS FOR CONCENTRATING AN ANALYTE**

(71) Applicant: BSH Hausgeräte GmbH, 81739 München (DE)
(72) Inventor: Cheong, Yi Heng, 440031 Singapore (SG); Chia, Liping Sharon, 141079 Singapore (SG); Sagar, Kaushal, 573943 Singapore (SG)

(57) **Abstract**

A preconcentration apparatus (100) is described, which is configured to increase the concentration of an analyte within a liquid solution. The apparatus (100) comprises a housing (101, 102, 202) which encloses an interior that is configured to take up a sample medium (105) imbibed with the liquid solution of the analyte. The apparatus (100) is configured to generate a flow of gaseous fluid (206) through the interior of the housing (101, 102, 202) towards an output opening (103) of the housing (101, 102, 202), such that the liquid solution is moved within the sample medium (105) towards a concentration area (300, 303) of the sample medium (105), which is located at the output opening (103) of the housing (101, 102, 202).

## Description

The present document relates to a method and a corresponding apparatus, which are directed at increasing the concentration of an analyte within a liquid solution.

Natural or treated water may comprise contaminants such as microplastics and/or perfluoroalkyl substances (PFAS). Monitoring the quality of water, notably of drinking water, typically involves measuring the concentration of such contaminants within the water. The concentration of a contaminant may be relatively low, and may therefore be difficult to measure.

Hence, it may be beneficial to increase the concentration of a contaminant, i.e., of an analyte, within the water, i.e., within a liquid solution, in a defined way, thereby enabling a precise measurement of the concentration. The present document addresses the technical problem of increasing the concentration of an analyte within a liquid solution in a defined way, without impacting the stability of the analyte. The technical problem is solved by each one of the independent claims. Preferred examples are described in the dependent claims.

According to an aspect, a preconcentration apparatus is described, which is configured to increase the concentration of an analyte within a liquid solution. Example analytes are a contaminant such as a microplastic, a pesticide and/or a PFAS. The liquid solution may comprise water.

The apparatus comprises a housing which encloses an interior that is configured to take up a sample medium imbibed with the liquid solution of the analyte. The sample medium may be configured to guide liquid (notably towards the output opening of the housing). The sample medium may comprise fibers and/or may comprise a fibrous material. In particular, the sample medium may comprise cellulose.

The apparatus is configured to generate a flow of gaseous fluid (e.g., of air) through the interior of the housing towards the (possibly single) output opening of the housing, such that the liquid solution is moved within the sample medium towards a concentration area of the sample medium, wherein the concentration area is located at (e.g., directly adjacent to and/or facing) the output opening of the housing. The concentration area may correspond to 10% or less of the surface of the sample medium.

The liquid solution may be pushed by the gaseous fluid towards the output opening of the housing. At the output opening the liquid may evaporate, thereby leaving behind the analyte within the sample medium. This process may be continued for a certain (possibly predefined) time duration, notably until a substantial fraction (e.g., 90% or more) of the liquid solution has evaporated from the sample medium.

As a result of this, a substantial increase of the concentration of the analyte may be achieved by
- concentrating the liquid solution within a relatively small concentration area; and
- evaporating the liquid from the liquid solution.

The housing typically comprises one or more input openings which are each configured to guide gaseous fluid into the interior of the housing, whereas the (single) output opening is configured to guide gaseous fluid out of the interior of the housing. The one or more input openings are each located at a remote and/or opposite side of the housing with respect to the output opening. As a result of this, a flow of gaseous fluid through the entire sample medium is caused, thereby further increasing the concentration of the analyte.

The apparatus may comprise a vacuum pump which is configured to suck gaseous fluid through the output opening out of the interior of the housing, thereby increasing the concentration of the analyte in a particularly reliable manner.

The housing is preferably configured to apply a pressure onto the sample medium which is placed within the interior of the housing. As a result of this, the sample medium is prevented from being moved by the flow of gaseous fluid. Furthermore, the flow of gaseous fluid is forced to flow through the sample medium. By doing this, the concentration of the analyte may be increased in a particularly reliable manner.

The housing may comprise a first plate and a second plate, which are located on opposite sides of the sample medium. The two plates may be pressed towards each other, to apply a pressure onto the sample medium. The first plate and the second plate may each be made up of acrylic. The housing may comprise one or more clamps configured to press the first plate and the second plate towards each other. The output opening may be implemented as a hole within the first plate. The one or more input openings may be located at the edge of the first plate and of the second plate. Furthermore, the one or more input openings may be formed by the gap between the first plate and the second plate. As a result of this, a particularly efficient and reliable preconcentration apparatus may be provided.

According to a further aspect, a method for increasing the concentration of an analyte within a liquid solution is described. The method comprises placing a sample medium imbibed with the liquid solution into the interior of a housing. Furthermore, the method comprises generating a flow of gaseous fluid through the interior of the housing towards an output opening of the housing, such that the liquid solution is moved within the sample medium towards a concentration area of the sample medium, which is located at the output opening of the housing. The method may further comprise causing a pressure onto the sample medium (e.g., using a first plate and a second plate of the housing), such that the sample medium is prevented from being moved by the flow of gaseous fluid, and/or such that the flow of gaseous fluid is forced to flow through the sample medium.

It should be noted that the methods and systems including its preferred embodiments as outlined in the present document may be used stand-alone or in combination with the other methods and systems disclosed in this document. In addition, the features outlined in the context of an apparatus are also applicable to a corresponding method. Furthermore, all aspects of the method and apparatus outlined in the present document may be arbitrarily combined. In particular, the features of the claims may be combined with one another in an arbitrary manner.

The invention is explained below in an exemplary manner with reference to the accompanying drawings, wherein
- Figure 1: illustrates a housing for a sample medium of the analyte solution, wherein the housing comprises an output opening at a concentration area of the sample medium;
- Figure 2: shows the use of a vacuum pump for generating a flow of a gaseous drying fluid through the interior of the housing;
- Figure 3: illustrated the sample medium with an increased concentration of the analyte at the concentration area; and
- Figure 4: shows a flow chart of an example method for increasing the concentration of an analyte.

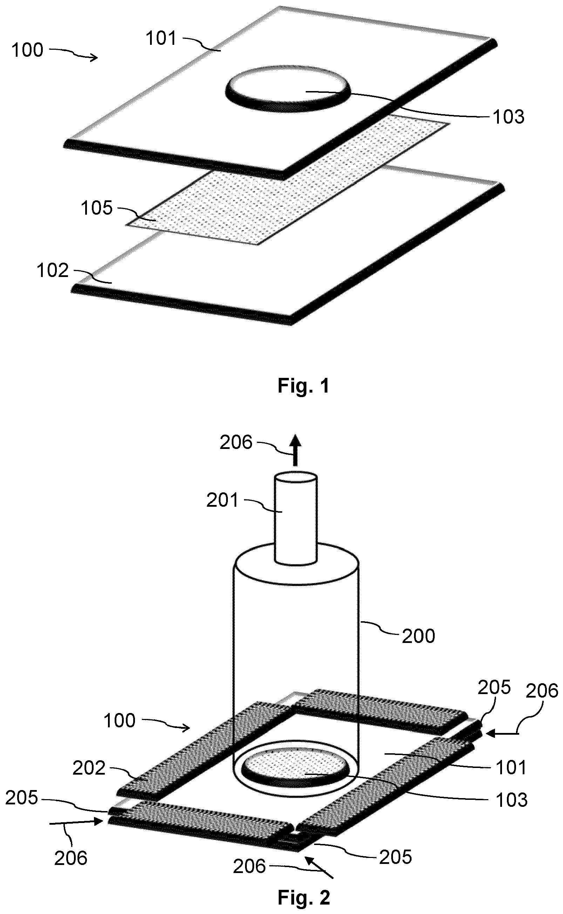

As outlined above, the present document is directed at increasing the concentration of an analyte within an analyte and/or liquid solution in an efficient, precise and deterministic manner. In this context, Fig. 1 shows an example apparatus 100 which is configured to increase the concentration of an analyte in a pre-determined concentration area of a sample medium 105. The sample medium 105 is configured to take up the analyte solution, such that the analyte solution can move and/or flow around within the sample medium 105. The sample medium 105 may e.g., comprise cellulose.

The apparatus 100 comprises plates 101, 102, in between which the sample medium 105 may be placed. In particular, the apparatus 100 comprises a first (and/or upper) plate 101 and a second (and/or lower) plate 102. The first plate 101 comprises an output opening 103, wherein the output opening 103 is positioned at the concentration area of the sample medium 105, i.e., the area of the sample medium 105, within which the concentration of the analyte is increased by the apparatus 100. The location of the output opening 103 within the first plate 101 defines the location of the concentration area. In particular, the location of the concentration area corresponds to the location of the output opening 103. The first and second plate 101, 102 form a housing of the apparatus 100, for taking up the sample medium 105.

As illustrated in Fig. 2, the first and the second plate 101, 102 may be coupled with one another using one or more sealing clamps 202. The sealing clamps 202 may be placed around the edges of the first and the second plate 101, 102. Furthermore, the sealing clamps 202 may be configured to press the first and the second plate 101, 102 together, such that the sample medium 105 is held in a defined and stable position and/or such that there is substantially no gap between the first plate 101 and the sample medium 105 and between the second plate 102 and the sample medium 105. The sealing clamps 202 at the edges of the first and the second plate 101, 102 may be part of the housing of the apparatus 100, for taking up the sample medium 105.

The sealing clamps 202 may have a reduced length compared to the respective edges of the first and the second plate 101, 102, such that at the corners of the plates 101, 102 input openings 205 are created, wherein the input openings 205 allow a gaseous drying fluid 206, such as air, to enter the gap between the first and the second plate 101, 102. Hence, the housing of the apparatus 100 comprises input openings 205 which are configured to let a gaseous drying fluid 206 enter the interior of the housing, in which the sample medium 105 is located.

The apparatus 100 shown in Fig. 2 is configured to generate a flow of gaseous drying fluid 205 through the sample medium 105 that is located between the two plates 101, 102. The flow of drying fluid 205 enters the sample medium 105 at remote ends (e.g., at the corners and/or the edges of the sample medium 105) and traverses the sample medium 105 towards the concentration area, before leaving the sample medium 105 at the concentration area of the sample medium 105 (wherein the concentration area is located below the output opening 103 of the first plate 101). In the example shown in Fig. 2, the flow of drying fluid 206 is generated using a vacuum pump (not shown) which is coupled to a vacuum nozzle 200 that is placed above the output opening 103 of the first plate 101. The vacuum pump may be coupled to the vacuum nozzle 200 using a connector 201.

Using the vacuum pump, the drying fluid 206 (e.g., air) is sucked through the output opening 103 of the first plate 101, thereby creating a depression at the output opening 103 of the first plate 101. This depression causes gaseous drying fluid 206 and analyte solution to be sucked through the sample medium 105 towards the output opening 103, thereby increasing the amount of analyte solution at the concentration area (compared to the amount of analyte solution in the remote areas of the sample medium 105). Furthermore, the depression at the output opening 103 and the flow of drying fluid out of the sample medium 105 and through the output opening 103 causes the liquid component of the analyte solution (e.g., water) to be evaporated at the concentration area, thereby gradually increasing the concentration of the analyte at the concentration area.

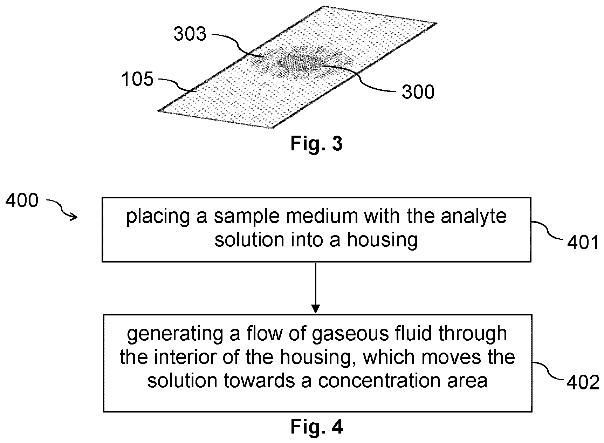

The flow of gaseous drying fluid 206 may be maintained for a certain time duration (e.g., until the sample medium 105 is substantially dry). Typically, increasing the time duration increase the concentration of the analyte within the concentration area, until reaching a certain saturation level. Fig. 3 shows a sample medium 105 subsequent to processing within the preconcentration apparatus 100. In can be seen that the concentration of analyte is increased within the opening area 303 of the sample medium 105, which corresponds to the output opening 103 of the first plate 101. Furthermore, the concentration area 300 is illustrated, which exhibits a particularly high concentration of the analyte. The concentration area 300 may be located at the center of the opening area 303.

Hence, a preconcentration method is described, which does not subject the liquid sample to relatively extreme conditions of temperature and/or voltage. A preconcentration medium 105 (such as cellulose) may be used, which is easily available. Preconcentration may be achieved with multiple different types of medium. Preconcentration of factors 80 to 170 times of the initial sample concentration may be achieved for sample analytes that are dissolved in liquid water. Samples of relatively low volume that contain a mix of solid and liquids can be preconcentrated using the preconcentration method which is described herein.

In the described preconcentration procedure a sample soaked cellulose medium 105 is sandwiched between two plates 101, 102. Subsequently, a vacuum is applied during the procedure. Fig. 3 shows the resulting preconcentrated sample analyte distribution.

The preconcentration method comprises the following steps. In a first step, a sample soaked cellulose substrate 105 is clamped between two acrylic sheets 101, 102, the top sheet 101 having a hole 103 in it. A vacuum, e.g., of pressure 80 kPa (-20 kPa compared to atmospheric pressure), is effected with a vacuum nozzle 200 for a certain time duration (e.g., 30 min) until complete evaporation of the liquid is achieved.

The (possibly cellulose) sample medium 105 may have one or more of the following roles:
- The sample medium 105 controls the direction into which the sample is able to flow.
- The sample medium 105 influences the directional flow of the liquid solution due to the orientation of (possibly cellulose) fibres.
- The (possibly cellulose) fibre spacing determines the filtration effectiveness of the sampling medium 105. Hence, the sample medium 105 may be configured to filter out (relatively large) components which are carried by the liquid solution.

The (possibly acrylic) plates 101, 102 may have one or more of the following roles:
- The plates 101, 102 keep the sampling medium 105 flat.
- The hole 103 in the top plate 101 restricts the flow of the liquid solution driven by air 206. This delineates the sample analyte preconcentration area 300, 303 within the sample medium 105.

The vacuum pump is the primary driver of the preconcentration and isolates the preconcentration area 300, 303 to a location within the sample medium 105, wherein the preconcentration area 300, 303 is delineated by the hole 103 in the plate 105. The pump may be configured to supplement a 80 kPa vacuum through the nozzle connection 200, 201 to the plate 101.

The (possibly) acrylic plates 101, 102 may have dimensions 15 by 10 cm and/or a thickness of 3 mm. The hole 103 in the top plate 101 may have a diameter of 16 mm. The diameter of the hole 103 is preferably narrower and/or smaller than the width of the sampling medium 105 to constrain all liquid flow to be within the sampling medium 105. Bull clips may be used as clamps 202 to constrain the sampling medium 105 to be flat and held between the plates 101, 102.

Fig. 4 shows a flow chart of an example method 400 for increasing the concentration of an analyte within a liquid solution. The method 400 comprises placing 401 a (fibrous) sample medium 105 which is imbibed with the liquid solution into the interior of a housing 101, 102, 202. The housing 101, 102, 202 may comprise two plates 101, 102 (one on each side of the sample medium 105), which are configured to apply a pressure onto the sample medium 105 (to hold the sample medium 105 in a fixed position).

The method 400 further comprises generating 402 a flow of gaseous fluid 206 (e.g., of air) through the interior of the housing 101, 102, 202 towards an output opening 103 of the housing 101, 102, 202, such that the liquid solution is moved within the (fibrous) sample medium 105 towards a concentration area 300, 303 of the sample medium 105, wherein the concentration area 300, 303 is located at the output opening 103 of the housing 101, 102, 202. The output opening 103 may be a hole in the first plate 101 of the housing 101, 102, 105.

As indicated above, a pressure may be caused onto the sample medium 105 using the first plate 101 and the second plate 102 of the housing 101, 102, 202. By doing this, the sample medium 105 is prevented from being moved by the flow of gaseous fluid 206. Furthermore, the flow of gaseous fluid 206 is forced to flow through the sample medium 105.

The liquid solution is moved towards the concentration area 303, 303. Furthermore, the liquid (e.g., the water) within the liquid solution evaporates at the concentration area 300, 303. As a result of this, the concentration of the analyte is increased in a localized manner within the concentration area 300, 303. The concentration area 300, 303 may represent 10% or less of the surface of the sample medium 105.

It should be noted that the description and drawings merely illustrate the principles of the proposed methods and systems. Those skilled in the art will be able to implement various arrangements that, although not explicitly described or shown herein, embody the principles of the invention and are included within its spirit and scope. Furthermore, all examples and embodiment outlined in the present document are principally intended expressly to be only for explanatory purposes to help the reader in understanding the principles of the proposed methods and systems. Furthermore, all statements herein providing principles, aspects, and embodiments of the invention, as well as specific examples thereof, are intended to encompass equivalents thereof.

## Claims

1. A preconcentration apparatus (100) which is configured to increase a concentration of an analyte within a liquid solution; wherein
- the apparatus (100) comprises a housing (101, 102, 202) which encloses an interior that is configured to take up a sample medium (105) imbibed with the liquid solution of the analyte; and
- the apparatus (100) is configured to generate a flow of gaseous fluid (206) through the interior of the housing (101, 102, 202) towards an output opening (103) of the housing (101, 102, 202), such that the liquid solution is moved within the sample medium (105) towards a concentration area (300, 303) of the sample medium (105), which is located at the output opening (103) of the housing (101, 102, 202).

2. The preconcentration apparatus (100) of claim 1, wherein
- the housing (101, 102, 202) comprises one or more input openings (205) which are each configured to guide gaseous fluid (206) into the interior of the housing (101, 102, 202);
- the output opening (103) is configured to guide gaseous fluid (206) out of the interior of the housing (101, 102, 202); and
- the one or more input openings (205) are each located at a remote and/or opposite side of the housing (101, 102, 202) with respect to the output opening (103).

3. The preconcentration apparatus (100) of any previous claim, wherein the apparatus (100) comprise a vacuum pump configured to suck gaseous fluid (206) through the output opening (103) out of the interior of the housing (101, 102, 202).

4. The preconcentration apparatus (100) of any previous claim, wherein the housing (101, 102, 202) is configured to apply a pressure onto the sample medium (105) which is placed within the interior of the housing (101, 102, 202), such that
- the sample medium (105) is prevented from being moved by the flow of gaseous fluid (206); and
- the flow of gaseous fluid (206) is forced to flow through the sample medium (105).

5. The preconcentration apparatus (100) of any previous claim, wherein the housing (101, 102, 202) comprises a first plate (101) and a second plate (102), which are located on opposite sides of the sample medium (105) and which are pressed towards each other, to apply a pressure onto the sample medium (105).

6. The preconcentration apparatus (100) of claim 5, wherein the housing (101, 102, 202) comprises one or more clamps (202) configured to press the first plate (101) and the second plate (102) towards each other.

7. The preconcentration apparatus (100) of any of claims 5 to 6, wherein the output opening (103) is a hole within the first plate (101).

8. The preconcentration apparatus (100) of any of claims 5 to 7, wherein the first plate (101) and the second plate (102) are each made up of acrylic.

9. The preconcentration apparatus (100) of any of claims 5 to 8, wherein the housing (101, 102, 202) comprises one or more input openings (205) which
- are located at an edge of the first plate (101) and of the second plate (102); and/or
- are formed by a gap between the first plate (101) and the second plate (102).

10. The preconcentration apparatus (100) of any previous claim, wherein
- the sample medium (105) is configured to guide liquid;
- the sample medium (105) comprises fibers and/or comprises a fibrous material; and/or
- the sample medium (105) comprises cellulose.

11. A method (400) for increasing a concentration of an analyte within a liquid solution; wherein the method (400) comprises
- placing (401) a sample medium (105) imbibed with the liquid solution into an interior of a housing (101, 102, 202); and
- generating (402) a flow of gaseous fluid (206) through the interior of the housing (101, 102, 202) towards an output opening (103) of the housing (101, 102, 202), such that the liquid solution is moved within the sample medium (105) towards a concentration area (300, 303) of the sample medium (105), which is located at the output opening (103) of the housing (101, 102, 202).

12. The method (400) of claims 11, wherein the method (400) comprises causing a pressure onto the sample medium (105) using a first plate (101) and a second plate (102) of the housing (101, 102, 202), such that
- the sample medium (105) is prevented from being moved by the flow of gaseous fluid (206); and
- the flow of gaseous fluid (206) is forced to flow through the sample medium (105).
